# FASCICULE DE BREVET EUROPEEN

(11) **EP 4 153 725 B1**
(45) Date de publication et mention de la délivrance du brevet: **04.12.2024**
(21) Numéro de dépôt: 21727860.5
(22) Date de dépôt: 21.05.2021
(51) Int. Cl.: A61F 2/00, A61L 27/36, A61L 27/56

(54) **BANDELETTE SOUS-URETRALE CONSTITUEE DE MATERIAU BIOLOGIQUE ISSU DE CORDON OMBILICAL**
SUBURETHRALE SCHLINGE AUS BIOLOGISCHEM MATERIAL AUS NABELSCHNUR
SUB-URETHRAL SLING CONSISTING OF BIOLOGICAL MATERIAL OBTAINED FROM UMBILICAL CORD

(30) Priorité: 21.05.2020 FR 2005394
(43) Date de publication de la demande: 29.03.2023
(73) Titulaire: TBF Genie Tissulaire (TBF), 69780 Mions (FR)
(72) Inventeur: BARNOUIN, Laurence, 38460 Venerieu (FR)
(74) Mandataire: Tripoz, Inès
(86) Numéro de dépôt international: PCT/EP2021/063627
(87) Numéro de publication internationale: WO 2021/234138

(56) Documents cités:
- WO-A1-2008/021391
- WO-A1-2008/021391
- US-A1- 2002 182 261
- US-A1- 2007 254 005
- US-B1- 9 855 301
- US-B1- 9 855 301
- LUCENA ROBERTO G. ET AL: "Experimental use of a cellulosic biopolymer as a new material for suburethral sling in the treatment of stress urinary incontinence", INTERNATIONAL BRAZ J UROL, vol. 41, no. 6, 1 December 2015 (2015-12-01), pages 1148 - 1153, XP055788292, Retrieved from the Internet <URL:https://www.scielo.br/pdf/ibju/v41n6/1677-5538-ibju-41-6-1148.pdf> DOI: 10.1590/S1677-5538.IBJU.2014.0155
- LUCENA ROBERTO G. ET AL: "Experimental use of a cellulosic biopolymer as a new material for suburethral sling in the treatment of stress urinary incontinence", INTERNATIONAL BRAZ J UROL, vol. 41, no. 6, 1 December 2015 (2015-12-01), pages 1148 - 1153, XP055788292, Retrieved from the Internet <URL:https://www.scielo.br/pdf/ibju/v41n6/1677-5538-ibju-41-6-1148.pdf> DOI: 10.1590/S1677-5538.IBJU.2014.0155
- LUCENA ROBERTO G. ET AL: "Experimental use of a cellulosic biopolymer as a new material for suburethral sling in the treatment of stress urinary incontinence", INTERNATIONAL BRAZ J UROL, vol. 41, no. 6, 1 December 2015 (2015-12-01), pages 1148 - 1153, XP055788292, Retrieved from the Internet <URL:https://www.scielo.br/pdf/ibju/v41n6/1677-5538-ibju-41-6-1148.pdf> DOI: 10.1590/S1677-5538.IBJU.2014.0155
- LUCENA ROBERTO G. ET AL: "Experimental use of a cellulosic biopolymer as a new material for suburethral sling in the treatment of stress urinary incontinence", vol. 41, no. 6, 1, 1 November 2015 (2015-11-01) - 1 December 2015 (2015-12-01), pages 1148 - 1153, XP055788292, Retrieved from the Internet <URL:https://www.scielo.br/pdf/ibju/v41n6/1677-5538-ibju-41-6-1148.pdf> [retrieved on 20210322], DOI: 10.1590/S1677-5538.IBJU.2014.0155

## Description

La présente invention est dans le domaine des dispositifs médicaux, notamment des dispositifs sous forme de biomatériaux pour le traitement de l'incontinence urinaire.

### ART ANTERIEUR

Les bandelettes sous-urétrales sont utilisées dans le traitement de l'incontinence urinaire chez la femme depuis plusieurs décennies. Il est estimé qu'une femme sur cinq souffre d'incontinence urinaire d'effort, et qu'environ 40000 bandelettes sous-urétrales sont posées par an en France.

La bandelette sous-urétrale est devenue le modèle standard pour soigner les incontinences urinaires d'effort insuffisamment soulagées par la rééducation périnéale.

Les fuites urinaires sont consécutives à un défaut de soutien ou à une altération du sphincter de la vessie. La pose de bandelettes vise à renforcer cette zone fragilisée.

Il existe aujourd'hui couramment deux types de bandelettes sous-urétrales, la bandelette TVT (Tension-free Vaginal Tape) et la bandelettes TOT (Trans-Obturator Tape).

La bandelette TVT mesure une trentaine de centimètres de long et est insérée en arrière du pubis. Elle est positionnée en U sous l'urètre. Les deux bras remontent de chaque côté et ressortent juste au-dessus de la vulve, au ras du pubis.

La bandelette TOT est disposée de façon plus horizontale que la bandelette TVT, elle traverse les muscles abducteurs des cuisses et ses extrémités émergent à la face interne de la cuisse sous le pli inguinal.

Les deux méthodes TVT et TOT sont efficaces, chacune présentant ses avantages ainsi que ses inconvénients.

La mise en place chirurgicale de ces deux types de bandelettes est couramment décrite dans la littérature.

Une nouvelle génération de bandelettes a été développée depuis 2017, les bandelettes dites "transmembranaires" (TMT) ou « mini-bandelettes ». Ces nouveaux dispositifs sont positionnés comme les bandelettes TOT mais au lieu de ressortir à l'extérieur, à la racine de la cuisse, ils s'ancrent à l'intérieur, dans le muscle obturateur interne.

Des bandelettes pour traiter l'incontinence chez l'homme existent également, environ 2000 de ces bandelettes sont posés par an en France.

Les bandelettes sous-urétrales peuvent être fabriquées à partir de deux types de matériaux, les matériaux synthétiques et les matériaux biologiques.

Différents types de matériaux synthétiques et organiques pour la fabrication de bandelettes sous-urétrales ainsi que leurs avantages et inconvénients sont divulgués dans Deval et al., J Gynecol Obstet Biol Reprod 2002 ; 31 : 131-143.

Concernant les matériaux biologiques, des auto, allo et xéno greffes ont été utilisées, telles que par exemple les greffes d'aponévrose antérieure des muscles droits de l'abdomen, de fascia lata, de derme, de derme de porc, de dure-mère. Globalement l'utilisation de ces matériaux biologiques ne s'est pas révélée efficace du fait d'un fort taux d'échec et de récidives pour certains, en cause notamment une élasticité moyenne voire une rigidité de ces greffes.

C'est pourquoi le matériau synthétique le plus couramment utilisé est du polypropylène tricoté monofilament ou multifilament car il induit peu de rejets et d'intolérances. Différents types de bandelettes synthétiques sont décrites dans la littérature. Néanmoins ces bandelettes synthétiques ne sont pas exemptes pour autant d'inconvénients et des complications après pose peuvent survenir, notamment en raison du gain en élasticité des matériaux synthétiques, ce gain étant lié aux mèches monofilaments.

Les complications peuvent être des saignements, des blessures ou érosions de la vessie, de l'urètre, du vagin, de la rétention urinaire etc. Ces taux de complications peuvent atteindre 10% des cas et sont parfois invalidantes.

Une remise en cause actuelle des bandelettes sous-urétrales ainsi qu'une absence d'alternatives aux matériaux couramment utilisés pour la fabrication, entraine le besoin d'un nouveau produit satisfaisant à la fois les exigences de qualité et d'efficacité pour le traitement de l'incontinence urinaire.

La demanderesse a mis au point un dispositif de bandelette sous-urétrale constituée d'un matériau biologique issu de cordon ombilical qui est un vaisseau ombilical et qui est exempt des inconvénients des dispositifs de l'art antérieur. La veine ombilicale est un vaisseau possédant de très bonnes propriétés mécaniques et élastiques et pouvant supporter des forces de traction en charge similaires aux bandelettes sous-urétrales existantes sur le marché, il est composé de fibres musculaires circulaires (tunica media) et d'une couche élastique (tunica intima).

Le document WO2008021391 divulgue un biomatériau comprenant une membrane de cordon ombilical et de la gelée de Wharton. Si le biomatériau peut comprendre au moins un vaisseau ombilical, aucun biomatériau consistant en un vaisseau ombilical n'est divulgué. Le biomatériau peut être utilisé dans le traitement de l'incontinence, il est utilisé comme complément à une procédure de fronde sous-urétrale pour assister la réparation du muscle gracilis. L'utilisation du matériau comme bandelette sous-urétrale n'est nullement suggérée, l'invention est axée sur la réparation tissulaire et l'utilisation de biomatériaux issus de cordon ombilical en remplacement du collagène. De plus la membrane de cordon ombilical ne possède pas les propriétés mécaniques nécessaires pour être utilisée comme bandelette sous-urétrale. Aucun biomatériau consistant en un vaisseau ombilical utilisé comme bandelette sous-urétrale n'est donc divulgué.

Le document US9855301 divulgue une greffe laminée fabriquée à partir d'un « birth tissue material ». Cette greffe comprend une composition placentaire morcelée pouvant comprendre du vaisseau ombilical placée entre deux membranes amniotiques. Aucun biomatériau consistant en un vaisseau ombilical n'est divulgué. Cette greffe peut être utilisée dans le traitement de l'incontinence urinaire sans mention de son rôle exact dans ledit traitement, de plus cette invention est axée sur la régénération tissulaire, la cicatrisation et l'utilisation comme greffe. L'utilisation de la greffe comme bandelette sous-urétrale n'est nullement suggérée et les membranes amniotiques utilisées pour la fabrication de la greffe ne possèdent pas les propriétés mécaniques nécessaires pour être utilisées comme bandelette sous-urétrale. Aucun biomatériau consistant en un vaisseau ombilical utilisé comme bandelette sous-urétrale n'est donc divulgué.

Le document US2007254005 divulgue une méthode de traitement de tissus implantables, ces tissus pouvant être issus de cordon ombilical. Sont divulgués des « tissue-based » patchs utilisés dans divers applications chirurgicales. Un support pubo-urétral pour le traitement de l'incontinence urinaire est divulgué. L'utilisation d'un vaisseau ombilical n'est néanmoins pas suggérée.

Dans l'article de LUCENA ROBERTO G. et al, International Braz J. Urol,Vol 41, n°6, 1, les résultats d'une étude des interactions entre une bandelette sous-urétrale en cellulose exopolysaccharide et les tissus de l'urètre après mise en place sont rapportés et ce document fait état de résultats satisfaisants. Est également divulguée dans ce document l'utilisation de SIS (small intestine submucosa) comme bandelette sous urétrale qui a conduit à une exceptionnelle biocompatibilité mais à une récurrence des problèmes d'incontinence.

Le document US2002/182261 A1 divulgue une matrice bio-polymère à base de tissu animal pouvant être utilisée en tant que fronde urétrale.

### DESCRIPTION DES FIGURES

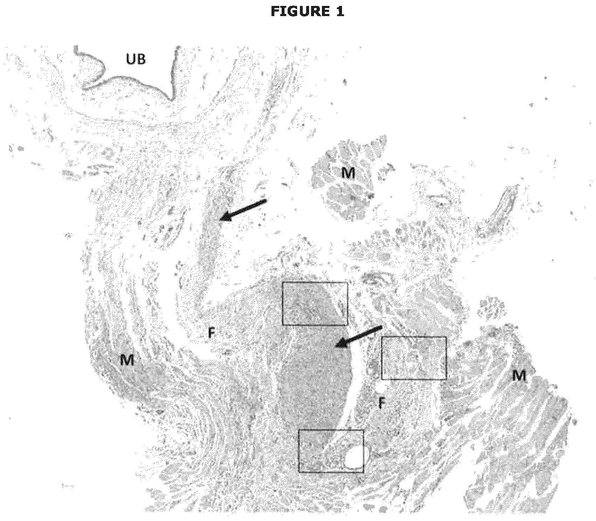

La Figure 1 présente une coupe histologique des tissus au site d'implantation du biomatériau chez une souris. Les flèches représentent le site d'implantation du biomatériau formant un coude sous l'urètre. F=Fibrose ; M=Fibres des muscles striés.

### DESCRIPTION DETAILLE DE L'INVENTION

La présente invention concerne une bandelette sous-urétrale constituée d'un matériau biologique issu de cordon ombilical consistant en un vaisseau ombilical ou un segment de celui-ci..

On entend par « vaisseau ombilical », un vaisseau sanguin, qui peut être la veine ou l'une des deux artères, les trois vaisseaux présents dans le cordon ombilical de mammifère étant exploitables selon l'invention. De préférence la veine ombilicale est utilisée.

On entend par « segment », une fraction du tissu d'origine, qui est un vaisseau sanguin, et qui a été sectionné en deux extrémités de telle sorte que la fraction présente la forme d'un cylindre.

Dans un mode de réalisation, le biomatériau issu de cordon ombilical selon l'invention est caractérisé en ce que ledit vaisseau ombilical ou segment de celui-ci est aplati ou collapsé.

Ledit au moins un vaisseau ombilical ou segment de celui-ci peut être aplati ou collapsé par n'importe quel procédé mécanique et/ou chimique connu de l'Homme du métier.

Dans un mode de réalisation, la bandelette sous-urétrale selon l'invention est caractérisée en ce que ledit vaisseau ombilical ou segment de celui-ci n'est pas retourné ou inversé.

Dans un mode de réalisation, la bandelette sous-urétrale selon l'invention est caractérisée en ce que ledit vaisseau ombilical ou segment de celui-ci est une veine ombilicale ou un segment de celle-ci.

Dans un mode de réalisation, la bandelette sous-urétrale selon l'invention est caractérisée en ce qu'elle est exempte de gelée de Wharton.

Dans un mode de réalisation, la bandelette sous-urétrale selon l'invention est caractérisée en ce qu'elle est exempte de membrane de cordon ombilical.

Dans un mode de réalisation, la bandelette sous-urétrale selon l'invention est caractérisée en ce qu'elle est exempte de membrane amniotique.

Dans un mode de réalisation, la bandelette sous-urétrale selon l'invention est caractérisée en ce qu'elle mesure de 3 à 50 cm de longueur.

Dans un mode de réalisation, la bandelette sous-urétrale selon l'invention est caractérisée en ce qu'elle mesure de 3 à 40 cm de longueur.

Dans un mode de réalisation, la bandelette sous-urétrale selon l'invention est caractérisée en ce qu'elle mesure de 3 à 30 cm de longueur.

Dans un mode de réalisation, la bandelette sous-urétrale selon l'invention est caractérisée en ce qu'elle mesure de 3 à 20 cm de longueur.

Dans un mode de réalisation, la bandelette sous-urétrale selon l'invention est caractérisée en ce qu'elle mesure de 5 à 25 mm de largeur.

Dans un mode de réalisation, la bandelette sous-urétrale selon l'invention est caractérisée en ce qu'elle mesure de 5 à 20 mm de largeur.

Dans un mode de réalisation, la bandelette sous-urétrale selon l'invention est caractérisée en ce qu'elle mesure de 5 à 15 mm de largeur.

Dans un mode de réalisation, la bandelette sous-urétrale selon l'invention est caractérisée en ce qu'elle possède un module de Young de 2 à 60 MPa.

Dans un mode de réalisation, la bandelette sous-urétrale selon l'invention est caractérisée en ce qu'elle possède un module de Young de 4 à 40 MPa.

Dans un mode de réalisation, la bandelette sous-urétrale selon l'invention est caractérisée en ce qu'elle possède un module de Young de 6 à 20 MPa.

Dans un mode de réalisation, la bandelette sous-urétrale selon l'invention est caractérisée en ce qu'elle résiste à une charge maximale de 10 à 100 N.

Dans un mode de réalisation, la bandelette sous-urétrale selon l'invention est caractérisée en ce qu'elle résiste à une charge maximale de 15 à 85 N.

Dans un mode de réalisation, la bandelette sous-urétrale selon l'invention est caractérisée en ce qu'elle résiste à une charge maximale de 20 à 70 N.

Le module de Young également appelé module d'élasticité ou module de traction, ainsi que la charge maximale ou résistance à la traction peuvent être mesurés par n'importe quelle technique connue de l'Homme du métier tel qu'un essai en traction.

Dans un mode de réalisation, la bandelette sous-urétrale selon l'invention est caractérisée en ce qu'elle est stérile.

Dans un mode de réalisation, la bandelette sous-urétrale selon l'invention est caractérisée en ce qu'elle est viro-inactivée.

Dans un mode de réalisation, la bandelette sous-urétrale selon l'invention est caractérisée en ce qu'elle est lyophilisée.

On entend par « lyophilisation », une technique visant à dessécher un produit préalablement surgelé par sublimation. Plus précisément, le liquide à ôter du produit est dans un premier temps transformé en glace par congélation ; puis par une dessiccation primaire, sous vide, la glace est sublimée ; enfin par une dessiccation secondaire, les molécules d'eau à la surface du produit sont extraites par désorption.

L'invention concerne également un procédé de préparation d'une bandelette sous-urétrale selon l'invention caractérisé en ce qu'il comprend les étapes de :
a) on dispose d'un vaisseau ombilical ou un fragment de celui-ci ;
b) on lyophilise ledit vaisseau ombilical ou fragment.

Dans un mode de réalisation, le procédé de préparation d'une bandelette sous-urétrale selon l'invention est caractérisé en ce qu'il comprend en outre entre l'étape a) et b) une étape de viro-inactivation.

On entend par « viro-inactivation », une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN.

Un procédé de viro-inactivation d'un guide implantable pour la régénération tissulaire comprenant un vaisseau ombilical est décrit dans le document WO2018083353.

Dans un mode de réalisation, le procédé de préparation d'une bandelette sous-urétrale selon l'invention est caractérisé en ce qu'il comprend en outre entre l'étape a) et b) une étape de viro-inactivation selon le procédé de viro-inactivation décrit dans WO2018083353.

Dans un mode de réalisation, le procédé de préparation d'une bandelette sous-urétrale selon l'invention est caractérisé en ce que ledit vaisseau ombilical ou segment de celui-ci est retourné ou inversé durant l'étape de viro-inactivation.

On entend par « vaisseau retourné ou inversé », que le tissu initial, qui est un vaisseau sanguin, est retourné sur lui-même de telle sorte que la paroi constitutive de la lumière du vaisseau avant retournement devient la paroi externe du vaisseau, et que la paroi qui était la paroi externe du vaisseau avant retournement devient la nouvelle paroi de la nouvelle lumière du vaisseau.

Retourner ledit vaisseau ombilical ou segment de celui-ci pendant l'étape de viro-inactivation permet de traiter également la paroi constitutive de la lumière du vaisseau de manière efficace. Eventuellement ledit vaisseau ombilical ou segment de celui-ci est « ré-inversé » pour retrouver sa configuration normale.

Dans un mode de réalisation, le procédé de préparation d'une bandelette sous-urétrale selon l'invention est caractérisé en ce qu'à l'étape b) ledit vaisseau ombilical ou segment de celui-ci est aplati durant le processus de lyophilisation.

L'aplatissement dudit vaisseau ombilical ou segment de celui-ci résulte notamment du collapsus de celui-ci suite au processus de lyophilisation.

Dans un mode de réalisation, le procédé de préparation d'une bandelette sous-urétrale selon l'invention est caractérisé en ce qu'à l'étape a) ledit vaisseau ombilical ou segment de celui-ci est une veine ombilicale ou un segment de celle-ci.

Dans un mode de réalisation, le procédé de préparation d'une bandelette sous-urétrale selon l'invention est caractérisé en ce qu'il comprend en outre ultérieurement à l'étape b) une étape de stérilisation.

Dans un mode de réalisation, le procédé de préparation d'une bandelette sous-urétrale selon l'invention est caractérisé en ce qu'il comprend en outre ultérieurement à l'étape b) une étape de stérilisation aux rayonnements gamma.

La bandelette sous-urétrale selon l'invention peut être utilisée pour traiter l'incontinence urinaire chez la femme et chez l'homme.

La présente invention concerne également une méthode de traitement de l'incontinence urinaire comprenant une étape d'implantation chez un sujet d'une bandelette sous-urétrale constituée d'un matériau biologique obtenu à partir d'un vaisseau ombilical ou un segment de celui-ci.

La bandelette sous-urétrale selon l'invention peut être utilisée pour traiter tout type d'incontinence urinaire chez la femme, telles que par exemple l'incontinence d'effort, l'incontinence par impériosité ou l'incontinence mixte résultant de la coexistence d'incontinence d'effort et d'impériosité. Il existe également d'autre types d'incontinences comme l'incontinence fonctionnelle due à des troubles psychiques, cognitifs ou métaboliques par exemple.

La bandelette sous-urétrale selon l'invention peut être utilisée selon la méthode TVT, TOT ou TMT sous forme de mini bandelette. De préférence elle est utilisée en méthode TOT ou TMT.

Les moyens de fixation de bandelette sous-urétrale selon l'invention peuvent être également les même que ceux utilisés pour les trois méthodes TVT, TOT ou TMT.

### EXEMPLES

### Exemple 1 : Exemple d'un mode de réalisation d'une bandelette sous-urétrale selon l'invention :

Un donneur proprement informé et consentant en accord avec les exigences de la Déclaration d'Helsinki offre en don le cordon ombilical issu d'un accouchement. Par exigence sanitaire relative aux dons de tissus et de cellules d'origine humaine, une qualification en amont du donneur est systématique. Cette qualification implique une recherche de virus HIV, hépatites B, C, HTLV et de la bactérie tréponème pâle responsable de la syphilis.

Le cordon ombilical est récupéré le plus tôt possible en salle d'accouchement. Il est avantageusement placé dans une boite stérile, comprenant une solution de NaCl à +4°C.

Au laboratoire, en salle stérile, la procédure suivante est appliquée :
Les vaisseaux sanguins du cordon ombilical sont identifiés et séparés les uns des autres par dissection.

La veine est découpée en un segment d'une longueur de 15 centimètres.

Le segment est rincé et hydraté dans des bains d'eau purifiée successifs, sous agitation douce, pendant 4 heures.

Le segment est congelé à sec à une température de -80°C.

Lors du traitement chimique, le segment est décongelé à l'air libre, à température ambiante, pendant une durée de 5 minutes. Cette étape de congélation, suivie d'une décongélation, assure une décellularisation importante du tissu biologique.

Le segment subit une succession de bains assurant un traitement chimique de celui-ci. Ce traitement a pour objectif une désinfection de la veine, et tout particulièrement sa viro-inactivation. Le segment est retourné sur lui-même puis ré-inversé durant le processus. Une agitation linéaire douce du milieu liquide est appliquée lors de chaque bain afin d'assurer une pénétration homogène des solvants dans les tissus.

Dans un premier temps, le segment est déposé dans un bain d'eau purifiée à température ambiante pendant environ 3 heures. Cette étape assure tant la fin de la décongélation du tissu physiologique, qu'une étape de lyse cellulaire par pression osmotique.

Puis, le segment est transféré dans un bain décontaminant composé d'éthanol 70 % v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, le segment est transféré dans un bain composé de peroxyde d'hydrogène à 30 % w/v à température ambiante pendant environ 15 minutes.

Puis, le segment est transféré dans un bain décontaminant composé de peroxyde d'hydrogène à 3 % w/v à température ambiante pendant environ 1 heure. Le segment obtenu est viro-inactivé.

L'action chimique appliquée au segment viro-inactivé est ensuite neutralisée, dans au moins un bain comportant de l'hydroxyde de sodium dilué autour d'un pH de 8,5. Le traitement d'au moins un bain de neutralisation s'effectue à température ambiante pendant environ 15 minutes.

Afin d'assurer un rééquilibrage du pH et pour éliminer au mieux les résidus organiques se détachant du tissu d'intérêt, le segment viro-inactivé est transféré dans au moins un bain de tampon physiologique (PBS), afin d'assurer son rééquilibrage physiologique. Le au moins un bain s'effectue à température ambiante pendant environ 15 minutes.

Enfin, le segment viro-inactivé est transféré dans deux bains successifs à l'eau purifiée, à température ambiante, pendant au moins 15 minutes et jusqu'à environ 1 heure.

A cette étape du procédé, on peut considérer que le segment obtenu selon ce traitement chimique est un segment en grande partie désinfecté, notamment viro-inactivé, et décellularisé.

Suite à cette partie relative aux traitements chimiques, le segment viro-inactivé subit une lyophilisation.

Le segment viro-inactivé est transféré dans un lyophilisateur, où une étape de congélation suivie d'une étape de lyophilisation sont effectuées selon les modalités suivantes :
- Congélation :
   ∘ La première étape de congélation s'effectue à une température d'acclimatation choisie pour ne pas endommager l'intégrité structurelle, fonctionnelle et biologique du segment viro-inactivé ;
   ∘ la deuxième étape de congélation s'effectue à la température finale de congélation qui est inférieure à la température d'acclimatation ;
- Lyophilisation :
   ∘ Une étape de lyophilisation primaire s'effectue par application d'un vide à environ 200 microbars et par application d'un profil de température ascendant ;
   ∘ Une étape secondaire de lyophilisation s'effectue par application d'un vide à environ 50 microbars et par application d'un profil de température descendant.

Suite à cette étape de lyophilisation, le segment est un segment désinfecté, notamment viro-inactivé, décellularisé, lyophilisé et aplati du fait du collapsus de celui-ci durant la lyophilisation.

Le segment est ensuite conditionné en conditions stériles, par exemple en double sachets conservable 5 ans à température ambiante.

Une dernière étape de stérilisation du segment est effectuée par exposition de celui-ci à des rayonnements gamma à 25-32 kGrays.

Le produit ainsi obtenu est prêt à l'utilisation comme bandelette sous-urétrale pour le traitement de l'incontinence.

### Exemple 2 : Utilisation d'un biomatériau issu de cordon ombilical selon l'invention comme bandelette sous-urétrale chez l'animal :

Un biomatériau issu de cordon ombilical selon l'invention obtenu par le procédé décrit à l'exemple 1 à l'inverse qu'il s'agit ici d'une artère ombilicale qui est plus petite et un segment plus court (2,5 à 4 cm de long) est utilisé pour pouvoir s'adapter à l'anatomie d'une souris femelle.

Pendant toute la procédure chirurgicale, les animaux ont été anesthésiés à l'isoflurane (3%). La chirurgie s'est déroulée comme suit :
Les nerfs pudendaux sont localisés dans la fosse ischiorectale. Une section bilatérale des nerfs pudendaux est réalisée. Environ 4 mm des nerfs sont retirés.

La paroi abdominale est ensuite ouverte et l'urètre est isolé du vagin.

Le biomatériau est placé sous l'urètre et chaque extrémité du biomatériau est fixée à la paroi abdominale pour étirer celui-ci.

La cavité abdominale est enfin suturée.

Les souris sont sacrifiées environ 1 mois après implantation du biomatériau, l'abdomen est ouvert par une incision médiane, la vessie et l'urètre sont exposés et des coupes histologiques sont réalisées. Tous les échantillons ont été coupés perpendiculairement à l'axe longitudinal de l'urètre.

Une coupe histologique au site d'implantation du biomatériau chez une des souris est présentée à la figure 1.

L'examen des sites d'implantation a révélé que les biomatériaux utilisés en tant que bandelettes sous-urétrales ont été relativement bien tolérées, suscitant une inflammation chronique minime à légère et une fibrose légère à modérée. Une partie de la réaction locale des tissus y compris la dégénérescence observée des fibres musculaires, a pu être provoquée par la procédure chirurgicale utilisée pour l'implantation.

### Exemple 3 : Essais mécaniques sur différents biomatériaux issus de cordon ombilical :

| | Résistance mécanique (MPa) | Force moyenne (N) | Déformation | (N) |
|---|---|---|---|---|
| Double membrane amniotique | 0,626 ± 0,397 | 4,43 ± 0,918 | 23+/- 6,2 % | 19 |
| Veine ombilicale | 0,367 ± 0,23 | 6,570 ± 3,808 | 12,2 +/- 2,4 % | 9 |

La veine ombilicale possède de meilleures caractéristiques en termes de résistance mécanique et de déformation qu'un biomatériau basé sur une ou deux membranes amniotiques à l'instar des inventions de WO2008021391 et US9855301.

## Revendications

1. Bandelette sous-urétrale **caractérisée en ce qu'**elle est constituée d'un matériau biologique issu de cordon ombilical consistant en un vaisseau ombilical ou un segment de celui-ci.

2. Bandelette sous-urétrale selon la revendication 1, **caractérisée en ce que** ledit vaisseau ombilical ou segment de celui-ci est aplati ou collapsé.

3. Bandelette sous-urétrale selon l'une quelconque des revendications précédentes, **caractérisée en ce que** ledit vaisseau ombilical ou segment de celui-ci est une veine ombilicale ou un segment de celle-ci.

4. Bandelette sous-urétrale selon l'une quelconque des revendications précédentes, **caractérisée en ce qu'**elle mesure de 3 à 50 cm de longueur.

5. Bandelette sous-urétrale selon l'une quelconque des revendications précédentes, **caractérisée en ce qu'**elle est viro-inactivée.

6. Bandelette sous-urétrale selon l'une quelconque des revendications précédentes, **caractérisée en ce qu'**elle est lyophilisée.

7. Procédé de préparation d'une bandelette sous-urétrale selon l'une quelconque des revendications précédentes, **caractérisé en ce qu'**il comprend les étapes de :
a) on dispose d'un vaisseau ombilical ou un segment de celui-ci ;
b) on lyophilise ledit vaisseau ombilical ou segment.

8. Procédé de préparation d'une bandelette sous-urétrale selon la revendication 7, **caractérisé en ce qu'**il comprend en outre entre l'étape a) et b) une étape de viro-inactivation.

9. Procédé de préparation d'une bandelette sous-urétrale selon l'une quelconque des revendications 7 et 8, **caractérisé en ce qu'**à l'étape b) ledit vaisseau ombilical ou segment de celui-ci est aplati durant le processus de lyophilisation.

10. Procédé de préparation d'une bandelette sous-urétrale selon l'une quelconque des revendications 7 à 9, **caractérisé en ce qu'**à l'étape a) ledit vaisseau ombilical ou segment de celui-ci est une veine ombilicale ou un segment de celle-ci.

## Patentansprüche

1. Suburethrales Band, **dadurch gekennzeichnet, dass** es aus einem biologischen Material besteht, das aus Nabelschnur gewonnen ist, bestehend aus einem Nabelschnurgefäß oder einem Teil davon.

2. Suburethrales Band nach Anspruch 1, **dadurch gekennzeichnet, dass** das Nabelschnurgefäß oder der Teil davon abgeflacht oder kollabiert ist.

3. Suburethrales Band nach einem der vorangehenden Ansprüche, **dadurch gekennzeichnet, dass** das Nabelschnurgefäß oder der Teil davon eine Nabelschnurvene oder ein Teil davon ist.

4. Suburethrales Band nach einem der vorangehenden Ansprüche, **dadurch gekennzeichnet, dass** es eine Länge von 3 bis 50 Zentimeter aufweist.

5. Suburethrales Band nach einem der vorangehenden Ansprüche, **dadurch gekennzeichnet, dass** es virusinaktiviert ist.

6. Suburethrales Band nach einem der vorangehenden Ansprüche, **dadurch gekennzeichnet, dass** es lyophilisiert ist.

7. Verfahren zum Herstellen eines suburethralen Bandes nach einem der vorangehenden Ansprüche, **dadurch gekennzeichnet, dass** es die Schritte umfasst:
a) Bereitstellen eines Nabelschnurgefäßes oder eines Teils davon;
b) Lyophilisieren des Nabenschnurgefäßes oder des Teils davon.

8. Verfahren zum Herstellen eines suburethralen Bandes nach Anspruch 7, **dadurch gekennzeichnet, dass** es weiterhin zwischen Schritt a) und Schritt b) einen Schritt der Virusinaktivierung umfasst.

9. Verfahren zum Herstellen eines suburethralen Bandes nach einem der Ansprüche 7 und 8, **dadurch gekennzeichnet, dass** in Schritt b) das Nabelschnurgefäß oder der Teil davon während des Lyophilisierens abgeplattet wird.

10. Verfahren zum Herstellen eines suburethralen Bandes nach einem der Ansprüche 7 bis 9, **dadurch gekennzeichnet, dass** in Schritt a) das Nabelschnurgefäß oder der Teil davon eine Nabelschnurvene oder ein Teil davon ist.

## Claims

1. Suburethral sling **characterized in that** it is made of a biological material from an umbilical cord consisting of an umbilical vessel or a segment thereof.

2. Suburethral sling according to claim 1, **characterized in that** said umbilical vessel or segment thereof is flattened or collapsed.

3. Suburethral sling according to any one of the preceding claims, **characterized in that** said umbilical vessel or segment thereof is an umbilical vein or a segment thereof.

4. Suburethral sling according to any one of the preceding claims, **characterized in that** it measures from 3 to 50 cm in length.

5. Suburethral sling according to any one of the preceding claims, **characterized in that** it is virus-inactivated.

6. Suburethral sling according to any one of the preceding claims, **characterized in that** it is lyophilized.

7. Method for preparing a suburethral sling according to any one of the preceding claims, **characterized in that** it comprises the steps of:
a) an umbilical vessel or a segment thereof is provided;
b) said umbilical vessel or segment is lyophilized.

8. Method for preparing a suburethral sling according to claim 7, **characterized in that** it further comprises between step a) and b) a virus-inactivation step.

9. Method for preparing a suburethral sling according to any one of claims 7 and 8, **characterized in that** in step b) said umbilical vessel or segment thereof is flattened during the lyophilization process.

10. Method of preparing a suburethral sling according to any one of claims 7 to 9, **characterized in that** in step a) said umbilical vessel or segment thereof is an umbilical vein or segment thereof.
